(19) Europäisches Patentamt
European Patent Office
Office européen des brevets

(11) Numéro de publication : **0 356 323 B1**

# (12) FASCICULE DE BREVET EUROPEEN

(45) Date de publication du fascicule du brevet : **30.09.92 Bulletin 92/40**

(51) Int. Cl.$^5$ : **C07D 401/06,** C07D 491/04, A61K 31/445

(21) Numéro de dépôt : **89402303.5**

(22) Date de dépôt : **18.08.89**

## (54) Dérivés de benzopyrrolidinone, leur procédé de préparation et les compositions pharmaceutiques les renfermant.

(30) Priorité : **19.08.88 FR 8811057**

(43) Date de publication de la demande : **28.02.90 Bulletin 90/09**

(45) Mention de la délivrance du brevet : **30.09.92 Bulletin 92/40**

(84) Etats contractants désignés : **AT BE CH DE ES FR GB GR IT LI LU NL SE**

(56) Documents cités :
**EP-A- 0 226 516**
**FR-A- 1 545 549**
**FR-A- 2 346 350**

(73) Titulaire : **ADIR ET COMPAGNIE**
**1 rue Carle Hébert**
**F-92415 Courbevoie Cédex (FR)**

(72) Inventeur : **Regnier, Gilbert**
**27 avenue du Plessis**
**F-92290 Chatenay Malabry (FR)**
Inventeur : **Dhainaut, Alain**
**7 rue des Guipières**
**F-78400 Chatou (FR)**
Inventeur : **Duhault, Jacques**
**14bis rue Paul Demange**
**F-78290 Croissy S/Seine (FR)**
Inventeur : **Lonchampt, Michel**
**80 rue H. Crette Clos de Ricbourg, Chevilly Larue**
**F-94150 Rungis (FR)**

(74) Mandataire : **Reverbori, Marcelle**
**Adir et Compagnie, 1, rue Carle Hébert**
**F-92415 Courbevoie Cédex (FR)**

EP 0 356 323 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet les nouveaux dérivés de benzopyrrolidinone, leur procédé de préparation et les compositions pharmaceutiques les renfermant.

Elle concerne particulièrement les dérivés de benzopyrrolidinone de formule générale I :

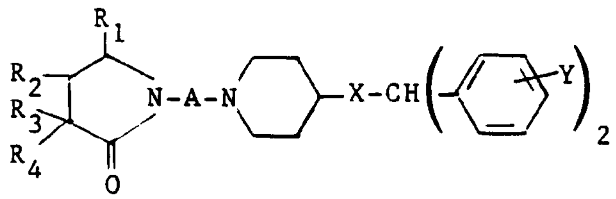

(I)

dans laquelle :

– $R_1$, $R_2$, $R_3$, $R_4$ ont les significations suivantes :

**a)**

. $R_1$ et $R_2$ représentent ensemble, avec la liaison carbone-carbone de l'hétérocycle auquel ils sont attachés, un noyau benzénique éventuellement substitué par un ou plusieurs atomes d'halogène tel que fluor ou chlore, ou par un ou plusieurs radicaux trifluorométhyle, alcoyle ou alcoxy ayant chacun de 1 à 4 atomes de carbone, ou méthylène dioxy ;

. et simultanément, $R_3$ et $R_4$, identiques ou différents, représentent chacun un atome d'hydrogène, un radical alcoyle ayant de 1 à 4 atomes de carbone en chaîne droite ou ramifiée, ou un radical cycloalcoyle ayant 5 ou 6 atomes de carbone ;

ou ;

**b)**

. $R_1$ représente un atome d'hydrogène, $R_4$ une liaison simple,

. et simultanément, $R_2$ et $R_3$ représentent ensemble avec la liaison carbone-carbone de l'hétérocycle auquel ils sont attachés un noyau benzénique éventuellement substitué par un ou plusieurs atomes d'halogène tel que fluor ou chlore, ou par un ou plusieurs radicaux trifluorométhyle, alcoyle ou alcoxy ayant chacun de 1 à 4 atomes de carbone, ou méthylène dioxy ;

de façon à former des structures appartenant à la famille de l'indole et de l'isoindole du type :

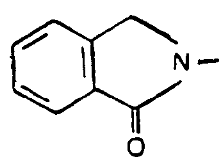 et 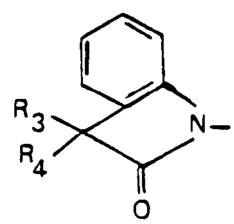

– **A** est une chaîne hydrocarbonée droite ou ramifiée ayant de 2 à 6 atomes de carbone éventuellement substituée par un radical hydroxy ;

– **X** représente une liaison simple, ou un atome d'oxygène ou de soufre ;

et

– **Y** représente un atome d'hydrogène ou d'halogène tel que fluor ou chlore ou un radical alcoyle ou alcoxy ayant chacun de 1 à 5 atomes de carbone.

L'état antérieur de la technique dans ce domaine est illustré notamment par le brevet français **2.346.350** qui concerne des composés de formule :

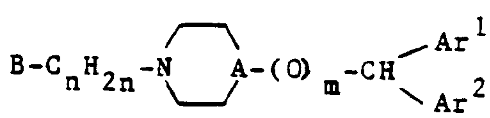

dans laquelle :

– $Ar^1$ et $Ar^2$ représentent, entre autres, un groupe phényle éventuellement substitué ;

– A représente :

>N- et simultanément m est zéro
ou,
>CH- et simultanément m est 1 ;
n est un nombre entier de 2 à 6, et
– B représente, entre autres, un radical de formule :

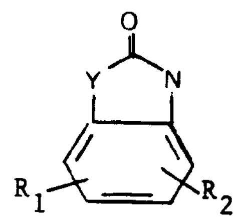

dans laquelle :
– $R_1$ et $R_2$ sont hydrogène, halogène, alcoyle inférieur ou trifluorométhyle, et
Y est oxygène, soufre ou azote substitué.

Ces dérivés de l'Etat de l'Art sont essentiellement des anti-histaminiques dont le produit leader est l'oxatomide, de formule :

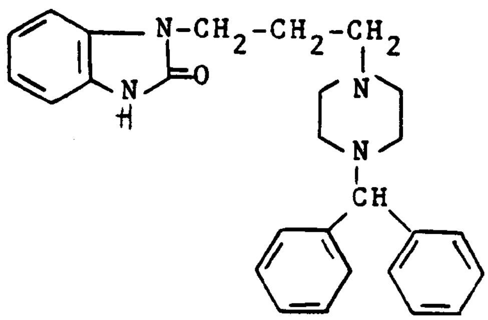

Le remplacement dans les dérivés de l'Etat de l'Art du groupe B par un groupement oxo indole ou oxo isoindole conduit aux dérivés de la présente invention qui se différencient des produits de l'état antérieur de la technique à la fois par leur structure chimique et par leur comportement pharmacologique.

La présente invention a également pour objet le procédé de préparation des dérivés de formule générale I caractérisé en ce que :
– l'on condense un dérivé de formule générale II :

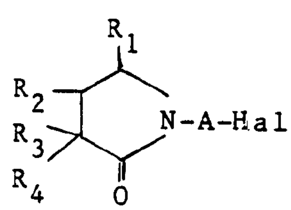

(II)

dans laquelle $R_1$, $R_2$, $R_3$, $R_4$ et A ont les significations précédemment définies, et Hal représente un atome d'halogène tel que chlore ou brome,
avec un dérivé de formule générale III :

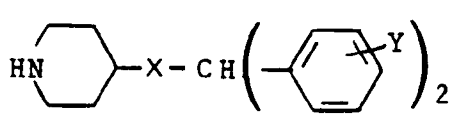

(III)

dans laquelle X et Y ont les significations précédemment définies.
La condensation des dérivés II et III est effectuée avantageusement dans un solvant polaire comme les

alcools ayant de 2 à 4 atomes de carbone, ou dans un solvant aprotique comme l'acétonitrile ou la méthyléthylcétone, à une température comprise entre 80 et 100°C en présence d'un accepteur de l'hydracide formé au cours de la réaction. Comme accepteur, on peut employer un excès du dérivé III, un carbonate alcalin comme $K_2CO_3$ ou $Na_2CO_3$ ou une amine tertiaire comme par exemple la triéthylamine.

Les produits de formule générale I sont des bases qui peuvent à ce titre former des sels avec des acides minéraux ou organiques biologiquement compatibles. Ces sels sont également inclus dans la présente invention.

Les produits de formule générale I peuvent être purifiés par chromatographie flash sur colonne de silice (35-70μ) sous pression de 0,5 à 1 bar, ou par cristallisation sous forme de sels dans des solvants appropriés.

Les matières premières utilisées pour préparer les dérivés de formule générale I sont regroupées dans les tableaux ci-après.

Tableau 1 :

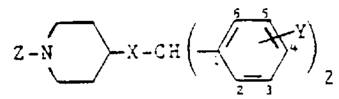

| Z | X | Y | Eb. (°C) | P.F.(°C) (cap) | Préparation rendement en % | méthode * |
|---|---|---|---|---|---|---|
| H | O | H | $156\text{-}167/_{0,2}$ | | 45 | ① |
| H | O | F(4) | $155\text{-}158/_{0,1}$ | | 46,2 | ② |
| $CH_3$ | S | H | $171\text{-}174/_{0,1}$ | | 78 | ③ |
| H | S | H | | 74-76 | 73 | ④ |

Méthodes de préparation :

①

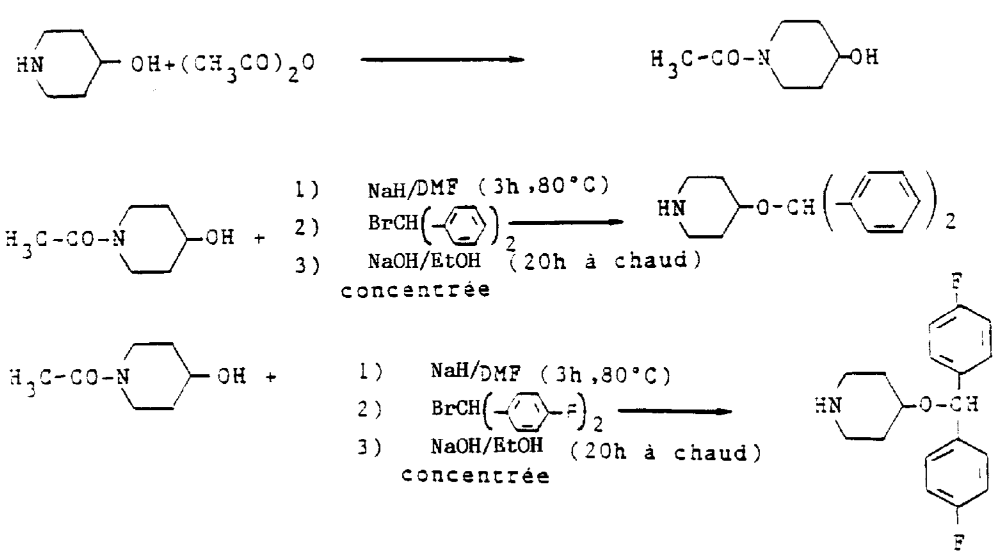


②

(3)

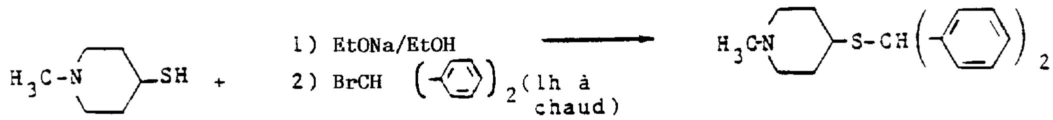


cf H. BARBERA, R.E. LYLE - J. Org. Chem., 27, 641 (1962)

(4)

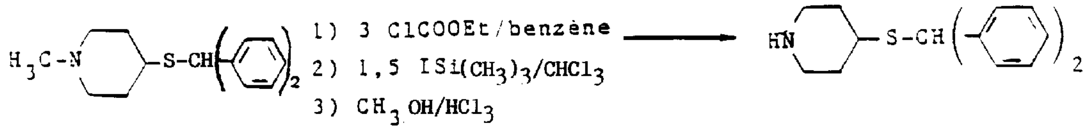


Selon la méthode de M.E. YUNG, M.A. LYSTER, J.C.S. Chem. Comm., 315 (1978).

Tableau 2

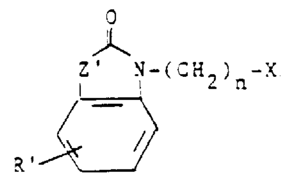

| Z' | X | R' | n | Eb (°C) | P.F. °C | Rendement (en % de préparation |
|---|---|---|---|---|---|---|
| O=C< | OH | H | 2 | | 148 (cap) | 90 |
| $CH_2$ | $OCOCH_3$ | H | 2 | huile | | 86 |
| $CH_2$ | Br | H | 2 | | 71 (cap) | 65 |
| >C=O | $OCH_3$ | H | 3 | huile | | 30 |
| $CH_2$ | Br | H | 3 | | 90 (cap) | 76 |
| >C=O | OH | (5) F | 2 | | 174 (K) | 76 |
| $CH_2$ | $OCOCH_3$ | (5) F | 2 | | 58 (K) | 29 |
| $CH_2$ | Br | (5) F | 2 | | 98-100 (cap) | 33 |
| >C=O | OH | (7) F | 2 | | 121-122 (cap) | 84 |
| $CH_2$ | $OCOCH_3$ | (7) F | 2 | | 69 (cap) | 95 |
| $CH_2$ | Br | (7) F | 2 | | 78 (cap) | 75 |
| >C=O | OH | (7) $CF_3$ | 2 | huile | | 64 |
| >C=O | OH | (5) $CF_3$ | 2 | | | |
| $(CH_3)_2C$ | $OCH_3$ | H | 3 | huile | | 71 |
| $(CH_3)_2C$ | Br | H | 3 | huile | | 75 |
| $(CH_3)_2C$ | $OCH_3$ | H | 2 | huile | | 50 |
| $(CH_3)_2C$ | Br | H | 2 | huile | | 33 |
| >C=O | $OCH_3$ | H | 2 | | 33-38 (cap) | 93 |
| 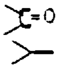 | $OCH_3$ | H | 2 | huile | | 70 |
| 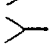 | Br | H | 2 | huile | | 84 |
| 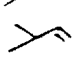 | $OCH_3$ | H | 3 | huile | | 41 |
| 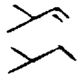 | $OCH_3$ | H | 3 | huile | | 95 |
| 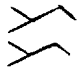 | Br | H | 3 | huile | | 90 |
| 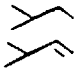 | $OCH_3$ | H | 2 | huile | | 39 |
|  | $OCH_3$ | H | 2 | huile | | 96 |
| 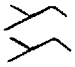 | Br | H | 2 | huile | | 69 |
| 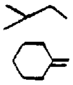 | $OCH_3$ | H | 2 | huile | | 60 |
| 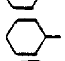 | $OCH_3$ | H | 2 | huile | | 94 |
| 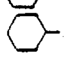 | Br | H | 2 | huile | | 91 |

Tableau 2 (suite)

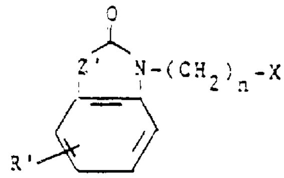

| Z' | X | R' | n | P.F. °C | Rendement (en %) de préparation |
|---|---|---|---|---|---|
| >C = O | $OCOCH_3$ | (5) F | 2 | 128 (K) | 29 |
| $CCl_2$ | $OCOCH_3$ | (5) F | 2 | | 68 |
| $CH_2$ | OH | (5) F | 2 | 103 (K) | 79 |
| $CH_2$ | Cl | (5) F | 2 | 130 (K) | 88 |
| CO | $OCH_3$ | (5) F | 3 | 76 (K) | 38 |
| $CCl_2$ | $OCH_3$ | (5) F | 3 | | 84 |
| $CH_2$ | $OCH_3$ | (5) F | 3 | | 68 |
| $CH_2$ | Br | (5) F | 3 | | 77 |

Tableau 3 :

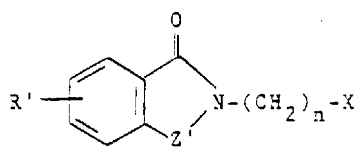

| Z' | X | R' | n | P.F.(°C) | Rendement (en % de préparation |
|---|---|---|---|---|---|
| $CH_2$ | OH | (5) F | 2 | 93-94 (cap) | 88 |
| $CH_2$ | Br | (5) F | 2 | 104-106(cap) | 75 |
| $CH_2$ | Br | (5) F | 3 | 63-66 (cap) | 55 |
| $CH_2$ | OH | (6) F | 2 | 105-107(cap) | 49 |
| $CH_2$ | Br | (6) F | 2 | 117-119(cap) | 72 |
| $CH_2$ | Br | 5,6di$OCH_3$ | 3 | 111 (cap) | 57 |

méthodes de préparation des matières premières de formule :

(A)

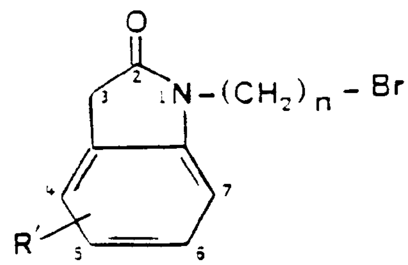

1) n = 2 R' = H

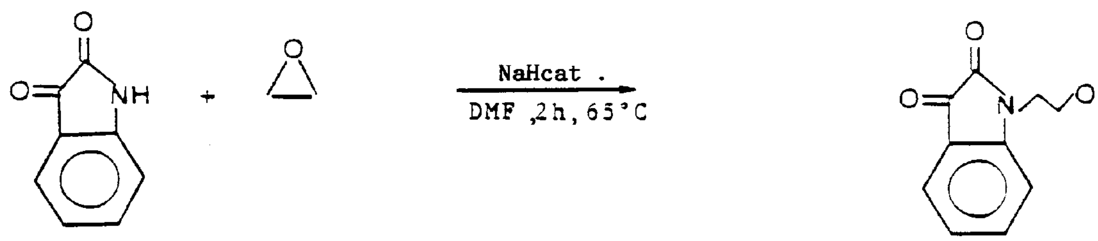


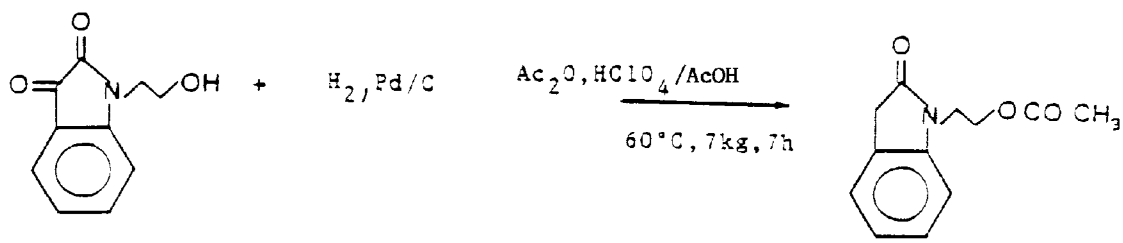


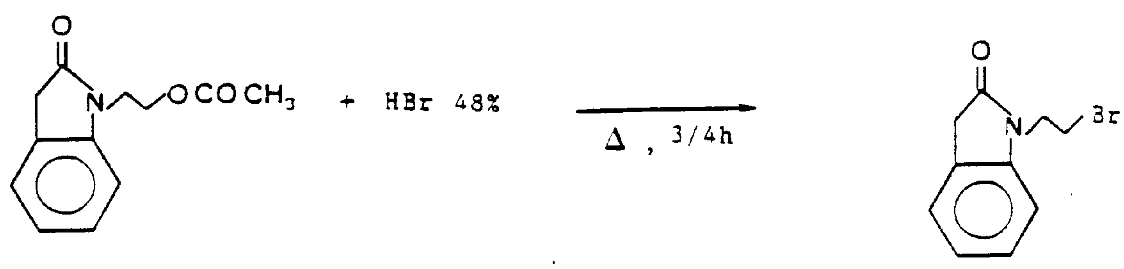


2) n = 3 R' = H

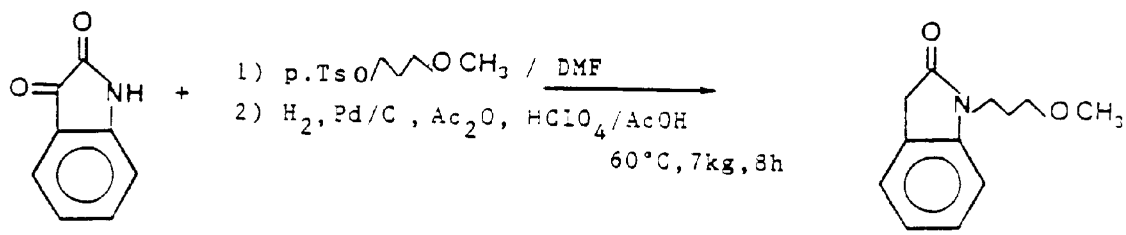

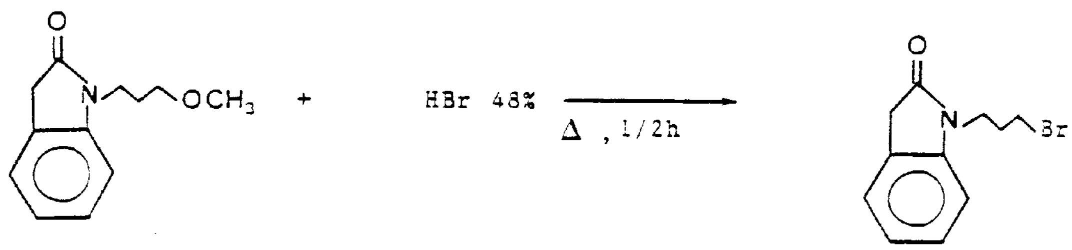


3) n = 2 R' = (5) F

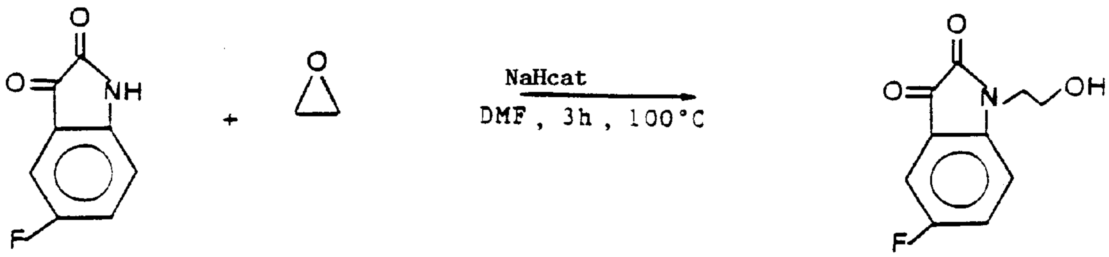


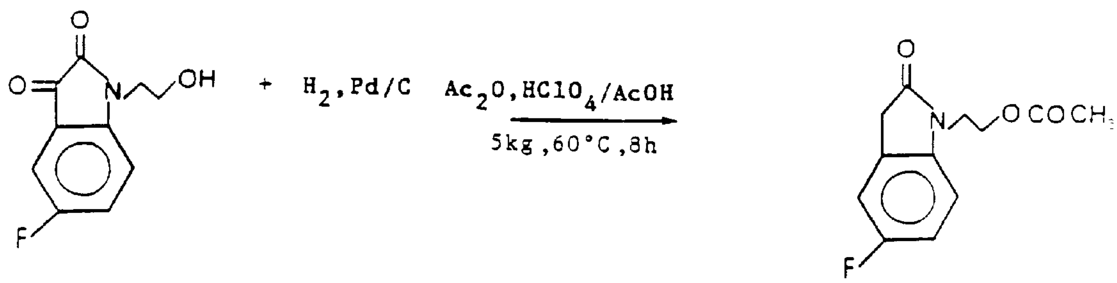


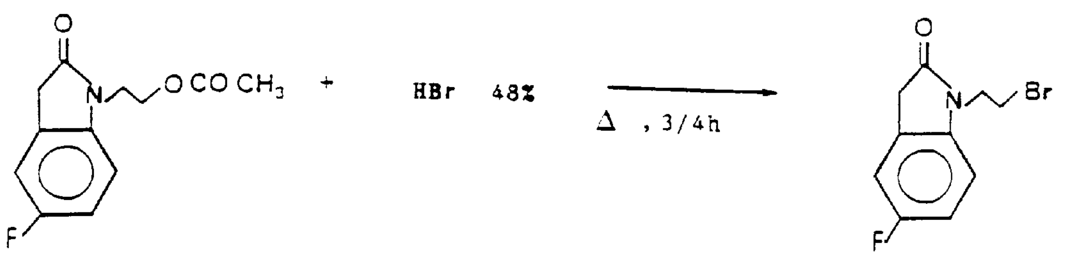


4) n = 2 R' =(7)F

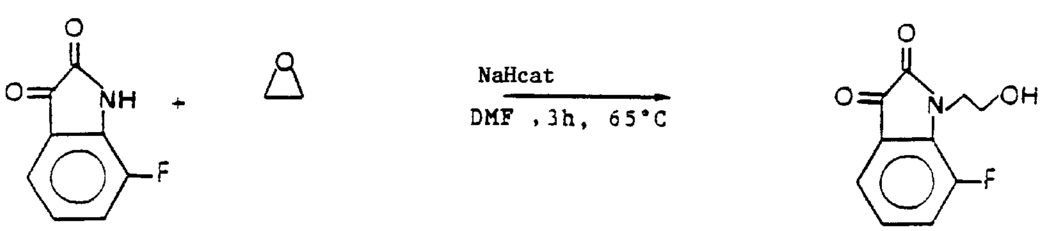

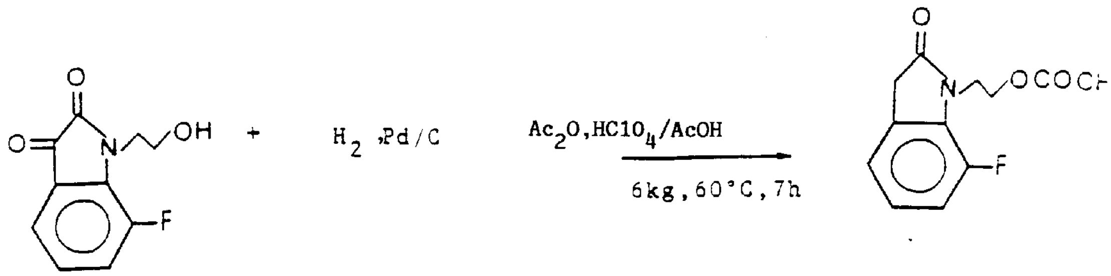
+
H2 ,Pd/C
Ac2O,HClO4/AcOH
6kg,60°C,7h

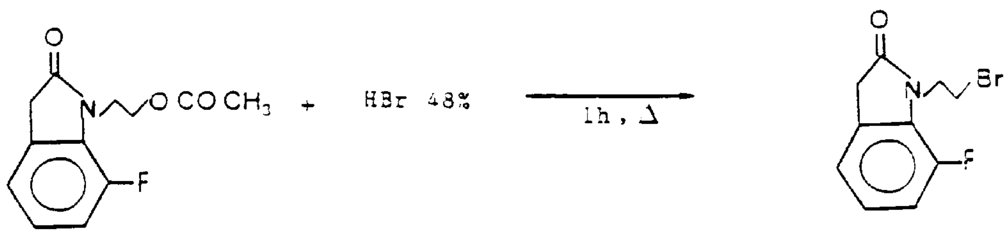
+
HBr 48%
1h, Δ

(B)

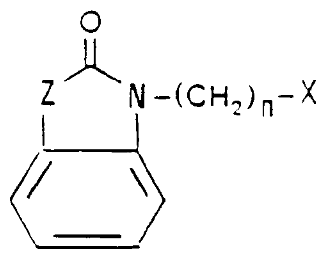

n = 2, X = $OCH_3$ :

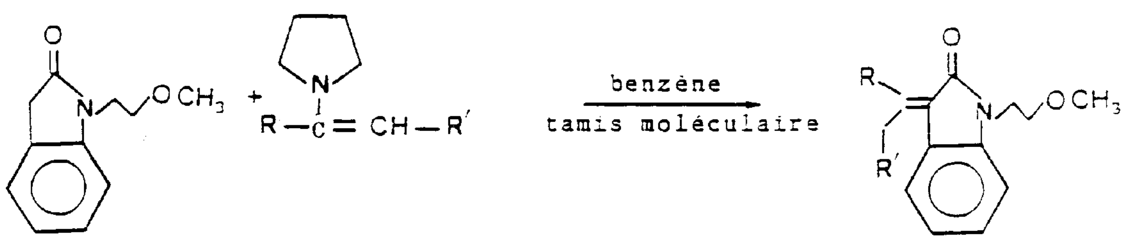
+
benzène
tamis moléculaire

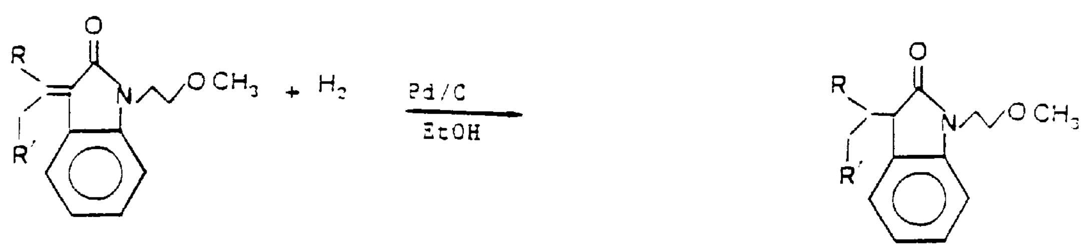
+ H2
Pd/C
EtOH

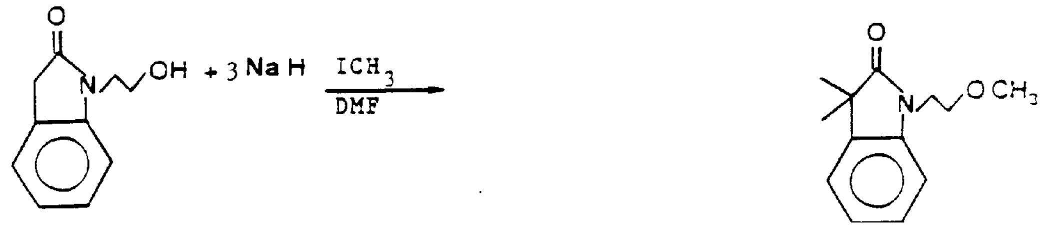


(C)

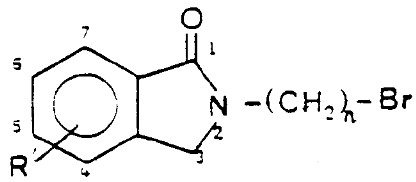

R'=H n=2

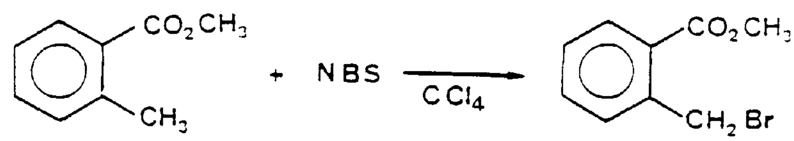


ne pas distiller

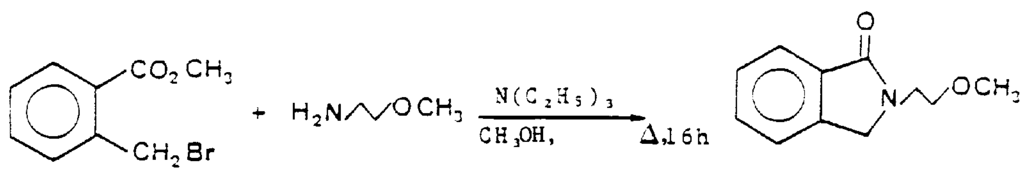


R=50%
P.E:140-144°C/0,1

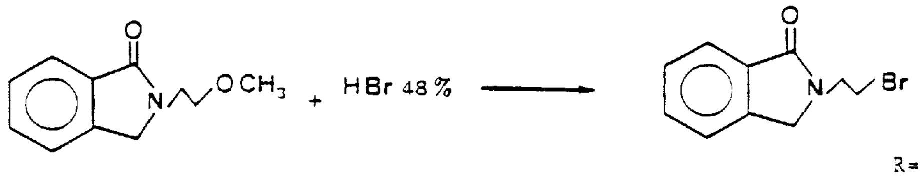


R=27%, P.F:89°C

R'=(5.6)di$OCH_3$ n=3

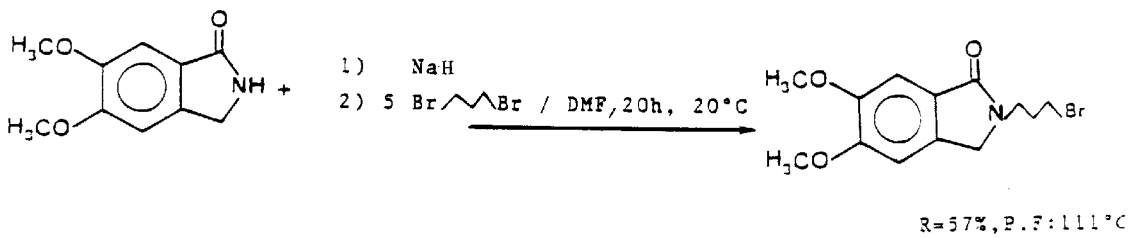


Les dérivés de formule générale I et leurs sels physiologiquement tolérables possèdent des propriétés pharmacologiques et thérapeutiques intéressantes, notamment des propriétés anti-bronchoconstrictrice, anti-allergique et anti-inflammatoire, qui de ce fait permettent leur utilisation comme médicament notamment dans le traitement des maladies bronchospastiques, des maladies inflammatoires aigües ou chroniques du tractus respiratoire associées ou non à des manifestations asthmatiformes d'origine allergique ou autres, des rhinites d'origines diverses et plus généralement des inflammations de la sphère ORL.

La présente invention a pour objet les compositions pharmaceutiques contenant comme principe actif un composé de formule générale I ou un de ses sels physiologiquement tolérables mélangé ou associé à un excipient pharmaceutique approprié comme par exemple, l'eau distillée, le glucose, le lactose, l'amidon, le talc, l'éthyl cellulose, le stéarate de magnésium ou le beurre de cacao.

Les compositions pharmaceutiques ainsi obtenues se présentent généralement sous forme dosée et peuvent contenir de 10 à 250mg de principe actif. Elles peuvent revêtir, par exemple, la forme de comprimés, dragées, gélules, suppositoires, solutions injectables ou buvables, et être selon les cas administrées par voie orale, rectale, parentérale ou locale à la dose de 10 à 250mg 1 à 3 fois par jour.

Les exemples suivants illustrent l'invention, les points de fusion étant, sauf mention contraire, déterminés au tube capillaire.

**Exemple 1 :**

(Benzhydryloxy-4 pipéridinoéthyl)-1 oxindole :

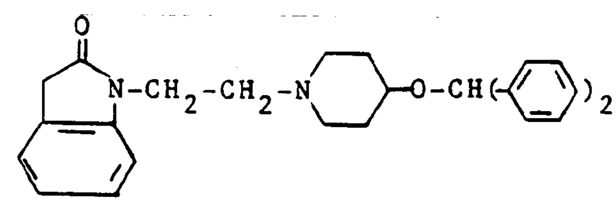

16g de benzhydryloxy-4 pipéridine (0,06 mole) et 7,2g de N-bromoéthyloxindole (0,03 mole) sont chauffés à reflux dans 230ml d'éthanol absolu pendant 16 heures.

Le mélange est concentré à sec, repris dans 300ml d'éther, lavé avec une solution aqueuse à 10% de $Na_2CO_3$, puis à l'eau. La solution organique est séchée sur $MgSO_4$ et concentrée. L'huile résiduelle est purifiée sur 450g de silice avec un mélange $CH_2Cl_2/CH_3COOC_2H_5$ (7/3) comme éluant. Le produit est cristallisé dans une solution éthanolique à 4% d'acide fumarique. On obtient le fumarate de benzhydryloxy-4 pipéridinoéthyl)-1 oxindole, P.F. : 153-154°C, (Rendement : 46%).

**Exemple 2 :**

(Benzhydryloxy-4 pipéridinoéthyl)-2 iso oxindole.

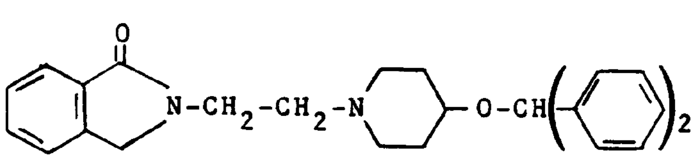

5,3g de benzhydryloxy-4 pipéridine (0,02 mole) et 2,4g de N-bromoéthyliso oxindole (0,01 mole) sont chauffés à reflux dans 80ml d'éthanol absolu pendant 16 heures.
Le mélange est concentré à sec, repris dans 100ml d'éther, lavé avec une solution aqueuse à 10% de $Na_2CO_3$, puis à l'eau. La solution organique est séchée sur $MgSO_4$ et concentrée. L'huile résiduelle est purifiée sur 250g de silice avec de l'acétate d'éthyle comme éluant. Le produit est cristallisé dans un mélange de $CH_2Cl_2$, $(C_2H_5)_2O$. On obtient ainsi le (benzhydryloxy-4 pipéridinoéthyl)-2 iso oxindole, P.F. : 128-129°C, (Rendement : 70%)

**Exemples 3 à 20 :**

Les produits ci-après exemplifiés ont été préparés selon le mode opératoire décrit dans l'exemple 1 :
**3)** (bis para-fluorobenzhydryloxy-4 pipéridinoéthyl)-1 oxindole, P.F. du fumarate acide correspondant : 164-167°C (éthanol/éther).
**4)** (bis para-fluorobenzhydryl-4 pipéridinopropyl)-1 oxindole, P.F. du maléate correspondant : 113°C (éthanol/éther).
**5)** (benzhydryloxy-4 pipéridinoéthyl)-1 oxindole, P.F. du fumarate correspondant : 160-161°C (éthanol).
**6)** (benzhydryloxy-4 pipéridinopropyl)-1 fluoro-7 oxindole, P.F. du fumarate correspondant : 134-137°C (éthanol).
**7)** (bis para-fluorobenzhydryloxy-4 pipéridinoéthyl)-1 oxindole, P.F. du fumarate correspondant : 172-173°C (éthanol).
**8)** (benzhydryloxy-4 pipéridinoéthyl)-1 fluoro-5 oxindole, P.F. du fumarate acide correspondant : 172-173°C (éthanol/éther).
**9)** (benzhydrylthio-4 pipéridinoéthyl)-1 oxindole, P.F. du fumarate correspondant : 177-179°C (éthanol).
**10)** (bis para-fluorobenzhydryl-4 pipéridinoéthyl)-1 oxindole, P.F. du maléate correspondant : 162°C (éthanol/éther).
**11)** (benzhydrylthio-4 pipéridinoéthyl)-2 iso oxindole.
**12)** (benzhydryloxy-4 pipéridinopropyl)-2 diméthoxy-5,6 iso oxindole, P.F.:49-54°C (éthanol/éther de pétrole).
**13)** (benzhydryloxy-4 pipéridinoéthyl)-2 méthylènedioxy-5,6 iso oxindole.
**14)** (benzhydryloxy-4 pipéridinoéthyl)-1 diméthyl-3,3 oxindole, P.F.:87-88°C (éthanol/éther de pétrole).
**15)** (benzhydryloxy-4 pipéridinoéthyl)-1 isopropyl-3 oxindole, P.F. du méthane sulfonate correspondant : 140-142°C (éthanol/eau).
**16)** (benzhydryloxy-4 pipéridinopropyl)-2 iso oxindole, P.F. (K) du chlorhydrate correspondant : 232°C (éthanol-éther).
**17)** (benzhydryloxy-4 pipéridinopropyl)-1 fluoro-5 oxindole, P.F. (K) du chlorhydrate correspondant : 230°C (éthanol).
**18)** (benzhydryloxy-4 pipéridinoéthyl)-2 fluoro-5 iso oxindole, P.F.: 138-140°C (éther).
**19)** (benzhydryloxy-4 pipéridinopropyl)-2 fluoro-5 iso oxindole, P.F.: 82-92°C (éthanol/eau/éther).
**20)** (benzhydryloxy-4 pipéridinoéthyl)-2 fluoro-6 iso oxindole, P.F.: 123-124°C (éther).

**Exemple 21 :**

Etude pharmacologique des dérivés de formule générale I :

Les dérivés de l'invention et le fenspiride produit du Prior Art pris comme substance de référence, ont été soumis à divers tests décrits dans **ARZNEIMITTEL - FORSCHUNG/DRUG RESEARCH 37 (II), 12, 1354-1355 (1987),** notamment ;

– au test de bronchoconstriction induite par l'histamine chez le cobaye,
– à la détermination du choc passif résultant de l'étude sur l'asthme expérimental induit chez le cobaye par des aérosols d'antigènes et
– à des essais in vitro sur l'inhibition de phosphodiestérase purifiée.

Les résultats obtenus sont répertoriés dans le tableau suivant.
L'examen de ce tableau montre l'intérêt des présents dérivés dont la faible toxicité et l'activité importante permettent leur utilisation en thérapeutique, notamment dans les domaines respiratoire et O R L.

| Composés des exemples | Toxicité $DL_{50}$ mg/kg souris | test d'Armitage cobaye P O<br>Dose mg/kg | <br>% de protection | choc passif cobaye $DE_{50}$ mg/kg | Inbihition Phosphodiestérase $IC_{50}$<br>basale | <br>+ Calmoduline |
|---|---|---|---|---|---|---|
| 1 | ≃ 200 (IP)<br>> 800 (PO) | 1 | + 66 | 2,5 (IP)<br>5 (PO) | $> 10^{-4}$M | $2.10^{-5}$M |
| 2 | > 800 (PO) | 1 | + 36 | 5(PO) | $> 10^{-4}$M | $3.10^{-5}$M |
| 3 | < 800 (PO) | 1 | + 47 | 20 (PO) | $> 10^{-4}$M | $3.10^{-6}$M |
| 4 | ≃ 200 (IP) | 20 | + 60 | 40 (IP) | $10^{-4}$M | $2.10^{-5}$M |
| 5 | 800 (PO) | 1 | + 72 | 5 (PO) | $> 10^{-4}$M | $10^{-4}$M |
| 6 | | 1 | + 36 | 20 (PO) | $> 10^{-4}$M | $10^{-4}$M |
| 7 | < 800 (PO) | 1 | + 46 | 5(PO) | $> 10^{-4}$M | $10^{-5}$M |
| 8 | > 800 (PO) | 1 | + 72 | 5(PO) | $> 10^{-5}$M | $7.10^{-6}$M |
| 9 | > 800 (PO) | 5 | + 60 | > 40 (PO) | $> 10^{-5}$M | $2.10^{-5}$M |
| 10 | > 800 (PO) | 5 | + 79 | 10 (PO) | $10^{-4}$M | $10^{-6}$M |

| Composés des exemples | Toxicité $DL_{50}$ mg/kg souris | test d'Armitage cobaye P O | | choc passif cobaye $DE_{50}$mg/kg | Inhibition Phosphodiestérase $IC_{50}$ | |
|---|---|---|---|---|---|---|
| | | Dose mg/kg | % de protection | | basale | + Calmoduline |
| 12 | 800 (PO) | 5 | + 79 | 20 (PO) | $7.10^{-5}$M | $2.10^{-5}$M |
| 14 | >800 (PO) | 5 | + 70 | >40 (PO) | $3.10^{-5}$M | $2.10^{-5}$M |
| 15 | >800 (PO) | 5 | + 50 | 5 (PO) | $10^{-4}$M | $3.10^{-5}$M |
| 16 | >800 (PO) | 5 | + 82 | 5 (PO) | >$10^{-4}$M | $3.10^{-5}$M |
| 17 | >800 (PO) | 5 | + 75 | 40 (PO) | >$10^{-4}$M | $10^{-5}$M |
| 18 | >800 (PO) | 5 | + 68 | 40 (PO) | $10^{-4}$M | $2.10^{-5}$M |
| 19 | >800 (PO) | 5 | + 68 | 10 (PO) | >$10^{-4}$M | $2.10^{-5}$M |
| 20 | >800 (PO) | 5 | + 43 | 40 (PO) | >$10^{-4}$M | $2.10^{-5}$M |
| Fenspiride | >800 (PO) | 2,5 | + 69 | 5 (IP)<br>10 (PO) | >$10^{-4}$M | >$10^{-4}$M |

Les dérivés de la présente invention ont également été testés sur l'oedème de l'oreille de souris, selon la technique de John M. YOUNG et al., Journal of Investigative Pharmacology, 82, 363-371 (1984). On a ainsi observé, qu'administrés P.O., à la dose de 36 micromole/kg, les dérivés de la présente invention, provoquent une diminution de l'oedème de l'oreille de souris allant de 15 à 50% selon les dérivés.

Dans les mêmes conditions et à la même dose :

– le fenspiride n'a aucune action ;

– l'oxatomide et le chlorhydrate d'[(oxo-2 benzimidazolinyl-3) éthyl]-1 benzhydryloxy-4 pipéridine - qui sont les deux produits de l'état antérieur de la technique structurellement les plus proches des dérivés de la présente invention - entrainent chacun une diminution de l'oedème de l'oreille de souris inférieure à 15% - prouvant ainsi la supériorité de dérivés de la présente invention sur les produits analogues antérieurement connus.

## Revendications

**Revendications pour les Etats contractants suivants : AT, BE, CH, DE, FR, GB, IT, LI, LU, NL, SE**

**1.** Les dérivés de benzopyrrolidinone de formule générale I :

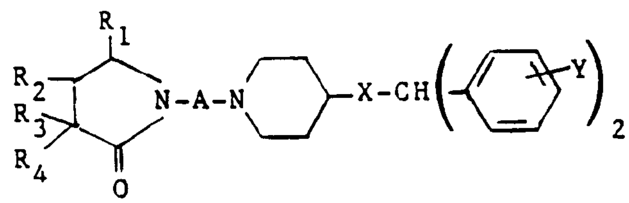

(I)

dans laquelle :

– $R_1$, $R_2$, $R_3$ et $R_4$ ont les significations suivantes :

**a)**

. $R_1$ et $R_2$ représentent ensemble, avec la liaison carbone-carbone de l'hétérocycle auquel ils sont attachés, un noyau benzénique éventuellement substitué par un ou plusieurs atomes d'halogène tel que fluor ou chlore, ou par un ou plusieurs radicaux trifluorométhyle, alcoyle ou alcoxy ayant chacun de 1 à 4 atomes de carbone, ou méthylène dioxy ;

. et simultanément, $R_3$ et $R_4$, identiques ou différents, représentent chacun un atome d'hydrogène, un radical alcoyle ayant de 1 à 4 atomes de carbone en chaîne droite ou ramifiée, ou un radical cycloalcoyle ayant 5 ou 6 atomes de carbone ;

ou ;

**b)**

. $R_1$ représente un atome d'hydrogène, $R_4$ une liaison simple,

. et simultanément, $R_2$ et $R_3$ représentent ensemble avec la liaison carbone-carbone de l'hétérocycle auquel ils sont attachés un noyau benzénique éventuellement substitué par un ou plusieurs atomes d'halogène tel que fluor ou chlore, ou par un ou plusieurs radicaux trifluorométhyle, alcoyle ou alcoxy ayant chacun de 1 à 4 atomes de carbone, ou méthylène dioxy ;

de façon à former des structures appartenant à la famille de l'indole et de l'isoindole du type :

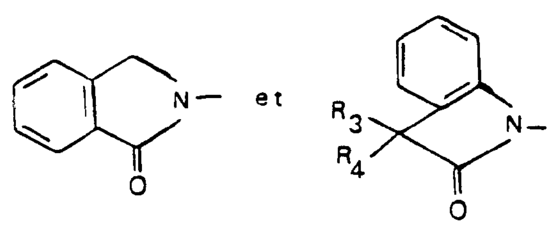

– **A** est une chaîne hydrocarbonée droite ou ramifiée ayant de 2 à 6 atomes de carbone éventuellement substituée par un radical hydroxy ;

– **X** représente une liaison simple, ou un atome d'oxygène ou de soufre ;

et

– **Y** représente un atome d'hydrogène ou d'halogène ou un radical alcoyle ou alcoxy ayant chacun de 1 à 5 atomes de carbone.

**2.** Les sels des dérivés de la revendication 1 avec des acides biologiquement compatibles.

**3.** le (benzhydryloxy-4 pipéridinoéthyl)-1 oxindole selon la revendication 1.

**4.** Le(benzhydryloxy-4 pipéridinoéthyl)-2 iso oxindole selon la revendication 1.

**5.** Le procédé de préparation des dérivés de la revendication 1 caractérisé en ce que l'on condense

– un dérivé de formule générale II :

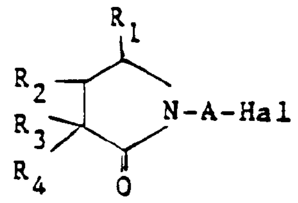

(II)

dans laquelle :

– $R_1$, $R_2$, $R_3$, $R_4$ et A ont les significations définies dans la revendication 1, et

– Hal représente un atome de chlore ou de brome,

– avec un dérivé de formule générale III :

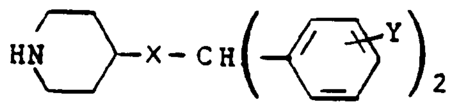

(III)

dans laquelle X et Y ont les significations définies dans la revendication 1.

**6.** Le procédé de préparation selon la revendication 5 caractérisé en ce que la condensation des dérivés II et III est effectuée dans un solvant polaire ou dans un solvant aprotique, à une température comprise entre 80 et 100°C, en présence d'un accepteur de l'hydracide formé au cours de la réaction.

**7.** Les compositions pharmaceutiques contenant comme principe actif un dérivé selon les revendications 1 à 4, avec des excipients pharmaceutiques appropriés.

**8.** Les compositions pharmaceutiques selon la revendication 7 présentées sous une forme convenant notamment pour le traitement des maladies bronchospastiques et des inflammations de la sphère O R L.

**Revendications pour les Etats contractants suivants: ES, GR**

**1.** Le procédé de préparation des dérivés de benzopyrrolidinone de formule générale I :

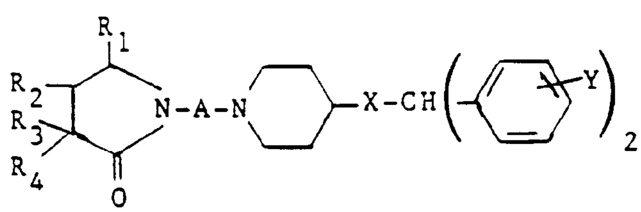

(I)

dans laquelle :

– $R_1$, $R_2$, $R_3$ et $R_4$ ont les significations suivantes :

**a)**

. $R_1$ et $R_2$ représentent ensemble, avec la liaison carbone-carbone de l'hétérocycle auquel ils sont attachés, un noyau benzénique éventuellement substitué par un ou plusieurs atomes d'halogène tel que fluor ou chlore, ou par un ou plusieurs radicaux trifluorométhyle, alcoyle ou alcoxy ayant chacun de 1 à 4 atomes de carbone, ou méthylène dioxy ;

. et simultanément, $R_3$ et $R_4$, identiques ou différents, représentent chacun un atome d'hydrogène, un radical alcoyle ayant de 1 à 4 atomes de carbone en chaîne droite ou ramifiée, ou un radical cycloalcoyle ayant 5 ou 6 atomes de carbone ;

ou ;

**b)**

. $R_1$ représente un atome d'hydrogène, $R_4$ une liaison simple,

. et simultanément, $R_2$ et $R_3$ représentent ensemble avec la liaison carbone-carbone de l'hétérocycle auquel ils sont attachés un noyau benzénique éventuellement substitué par un ou plusieurs atomes d'halogène tel que fluor ou chlore, ou par un ou plusieurs radicaux trifluorométhyle, alcoyle ou alcoxy ayant chacun de 1 à 4 atomes de carbone, ou méthylène dioxy ;

de façon à former des structures appartenant à la famille de l'indole et de l'isoindole du type :

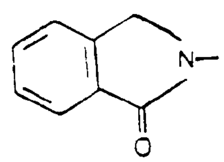

et

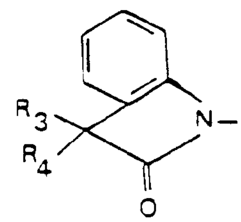

– **A** est une chaîne hydrocarbonée droite ou ramifiée ayant de 2 à 6 atomes de carbone éventuellement substituée par un radical hydroxy ;
– **X** représente une liaison simple, ou un atome d'oxygène ou de soufre ;
et
– **Y** représente un atome d'hydrogène ou d'halogène ou un radical alcoyle ou alcoxy ayant chacun de 1 à 5 atomes de carbone;
et de leurs sels avec des acides biologiquement compatibles;
caractérisé en ce que l'on condense :
– un dérivé de formule générale II :

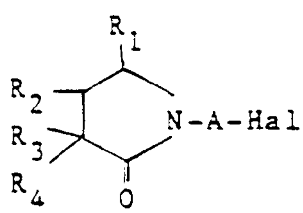

(II)

dans laquelle :

– $R_1$, $R_2$, $R_3$, $R_4$ and A ont les significations ci-dessus définies, et
Hal représente un atome de chlore ou de brome,

– avec un dérivé de formule générale III :

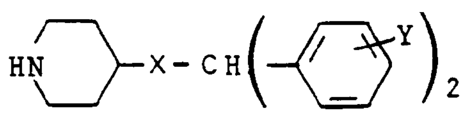

(III)

dans laquelle X et Y ont les significations précédemment définies ;
et, si on le désire, les produits de formule I ainsi obtenus sont traités avec des acides minéraux ou organiques biologiquement compatibles, pour donner les sels d'addition correspondants.

2. Le procédé de préparation selon la revendication 1 caractérisé en ce que la condensation des dérivés II et III est effectuée dans un solvant polaire ou dans un solvant aprotique, à une température comprise entre 80 et 100°C, en présence d'un accepteur de l'hydracide formé au cours de la réaction.

## Patentansprüche

## Patentansprüche für folgende Vertragsstaaten AT, BE, CH, DE, FR, GB, IT, LI, LU, NL, SE

1. Benzopyrrolidinon-Derivate der allgemeinen Formel I:

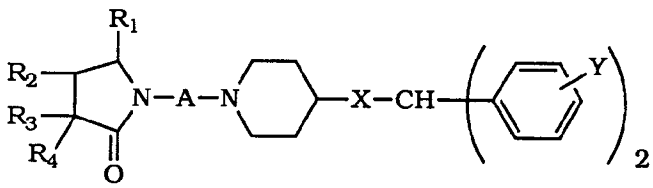

(I)

in der
– $R_1$, $R_2$, $R_3$ und $R_4$ die folgenden Bedeutungen besitzen:
a)
– $R_1$ und $R_2$ gemeinsam mit der Kohlenstoff-Kohlenstoff-Bindung des Heterocyclus, an den sie gebunden sind, einen Benzolkern, der gegebenenfalls durch eines oder mehrere Halogenatome, wie Fluor- oder Chloratome, oder durch eine oder mehrere Trifluormethylgruppen, Alkylgruppen oder Alkoxygruppen mit jeweils 1 bis 4 Kohlenstoffatomen substituiert ist, oder eine Methylendioxygruppe;
– und gleichzeitig $R_3$ und $R_4$, die gleichartig oder verschieden sind, jeweils Wasserstoffatome, geradkettige oder verzweigte Alkylgruppen mit 1 bis 4 Kohlenstoffatomen oder Cycloalkylgruppen mit 5 oder 6 Kohlenstoffatomen, bedeuten;
oder
b)
– $R_1$ ein Wasserstoffatom, $R_4$ eine Einfachbindung
– und gleichzeitig $R_2$ und $R_3$ gemeinsam mit der Kohlenstoff-Kohlenstoff-Bindung des Heterocyclus, an den sie gebunden sind, einen Benzolkern, der gegebenenfalls durch eines oder mehrere Halogenatome, wie Fluor- oder Chloratome, oder durch eine oder mehrere Trifluormethylgruppen, Alkylgruppen oder Alkoxygruppen mit jeweils 1 bis 4 Kohlenstoffatomen substituiert ist, oder eine Methylendioxygruppe bedeuten;
so daß sich Strukturen ergeben, die der Familie der Indole und der Isoindole des Typs

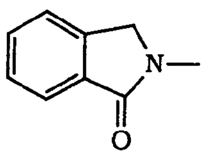

und

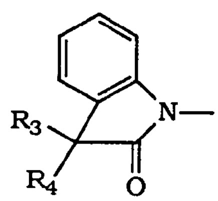

angehören; und
– A eine geradkettige oder verzweigte Kohlenwasserstoffkette mit 2 bis 6 Kohlenstoffatomen, die gegebenenfalls durch eine Hydroxygruppe substituiert ist;
– X eine Einfachbindung oder ein Sauerstoff- oder Schwefelatom; und
– Y ein Wasserstoffatom, ein Halogenatom oder eine Alkyl- oder Alkoxygruppe mit jeweils 1 bis 5 Kohlenstoffatomen
bedeuten.

2. Die Salze der Derivate nach Anspruch 1 mit biologisch vertraglichen Säuren.

3. 1-(4-Benzhydryloxy-piperidinoethyl)-oxindol gemäß Anspruch 1.

4. 2-(4-Benzhydryloxy-piperidinoethyl)-isooxindol gemäß Anspruch 1.

5. Verfahren zur Herstellung der Derivate nach Anspruch 1, **dadurch gekennzeichnet,** daß man ein Derivat der allgemeinen Formel II:

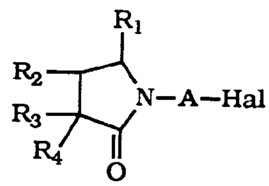

(II)

in der

- $R_1$, $R_2$, $R_3$, $R_4$ und A die in Anspruch 1 angegebenen Bedeutungen besitzen und
- Hal ein Chlor- oder Bromatom bedeutet;
  - mit einem Derivat der allgemeinen Formel III:

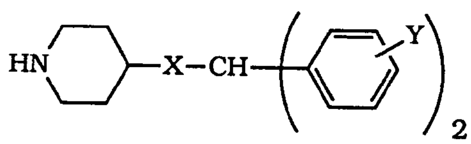

(III)

in der X und Y die in Anspruch 1 angegebenen Bedeutungen besitzen, kondensiert

.

6. Verfahren nach Anspruch 5, **dadurch gekennzeichnet**, daß die Kondensation der Derivate der Formeln II und III in einem polaren Lösungsmittel oder in einem aprotischen Lösungsmittel bei einer Temperatur zwischen 80 und 100°C in Gegenwart eines Akzeptors für die im Verlaufe der Reaktion gebildete Wasserstoffsäure durchgeführt wird.

7. Pharmazeutische Zubereitungen enthaltend als Wirkstoff ein Derivat nach einem der Ansprüche 1 bis 4 zusammen mit pharmazeutisch geeigneten Trägermaterialien.

8. Pharmazeutische Zubereitungen nach Anspruch 7 in einer Form, die insbesondere zur Behandlung von bronchospastischen Erkrankungen und Entzündungszuständen des Hals-Nasen-Ohren-Bereichs geeignet sind.

**Patentansprüche für folgende Vertragsstaaten: ES, GR**

1. Verfahren zur Herstellung der Benzopyrrolidinone-Derivate der allgemeinen Formel I:

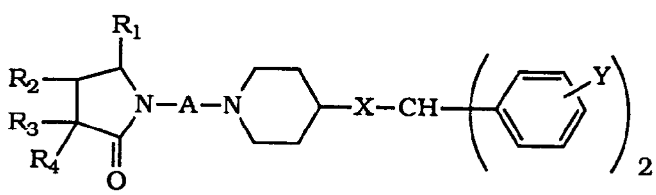

(I)

in der

- $R_1$, $R_2$, $R_3$ und $R_4$ die folgenden Bedeutungen besitzen:
  a)
  - $R_1$ und $R_2$ gemeinsam mit der Kohlenstoff-Kohlenstoff-Bindung des Heterocyclus, an den sie gebunden sind, einen Benzolkern, der gegebenenfalls durch eines oder mehrere Halogenatome, wie Fluor- oder Chloratome, oder durch eine oder mehrere Trifluormethylgruppen, Alkylgruppen oder Alkoxygruppen mit jeweils 1 bis 4 Kohlenstoffatomen substituiert ist, oder eine Methylendioxygruppe;
  - und gleichzeitig $R_3$ und $R_4$, die gleichartig oder verschieden sind, jeweils Wasserstoffatome, geradkettige oder verzweigte Alkylgruppen mit 1 bis 4 Kohlenstoffatomen oder Cycloalkylgruppen mit 5 oder 6 Kohlenstoffatomen, bedeuten;

  oder

b)

– $R_1$ ein Wasserstoffatom, $R_4$ eine Einfachbindung

– und gleichzeitig $R_2$ und $R_3$ gemeinsam mit der Kohlenstoff-Kohlenstoff-Bindung des Heterocyclus, an den sie gebunden sind, einen Benzolkern, der gegebenenfalls durch eines oder mehrere Halogenatome, wie Fluor- oder Chloratome, oder durch eine oder mehrere Trifluormethylgruppen, Alkylgruppen oder Alkoxygruppen mit jeweils 1 bis 4 Kohlenstoffatomen substituiert ist, oder eine Methylendioxygruppe bedeuten;

so daß sich Strukturen ergeben, die der Familie der Indole und der Isoindole des Typs

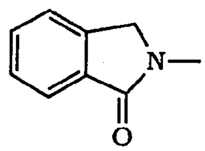

und

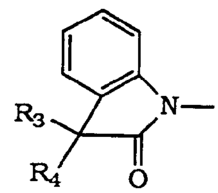

angehören; und

– A eine geradkettige oder verzweigte Kohlenwasserstoffkette mit 2 bis 6 Kohlenstoffatomen, die gegebenenfalls durch eine Hydroxygruppe substituiert ist;

– X eine Einfachbindung oder ein Sauerstoff- oder Schwefelatom; und

– Y ein Wasserstoffatom, ein Halogenatom oder eine Alkyl- oder Alkoxygruppe mit jeweils 1 bis 5 Kohlenstoffatomen bedeuten;

und deren Salzen mit biologisch verträglichen Sauren, **dadurch gekennzeichnet,** daß man

– ein Derivat der allgemeinen Formel II:

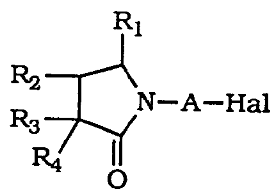

(II)

in der

– $R_1$, $R_2$, $R_3$, $R_4$ und A die oben angegebenen Bedeutungen besitzen und

– Hal ein Chlor- oder Bromatom bedeutet,

– mit einem Derivat der allgemeinen Formel III:

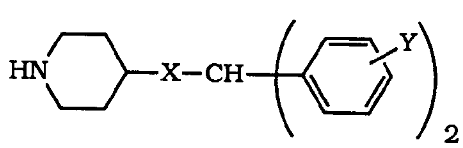

(III)

in der X und Y die oben angegebenen Bedeutungen besitzen; kondensiert und gewünschtenfalls die in dieser Weise erhaltenen Produkte der Formel I mit biologisch vertraglichen anorganischen oder organischen Sauren behandelt zur Bildung der entsprechenden Additionssalze.

**2.** Verfahren nach Anspruch 1, **dadurch gekennzeichnet,** daß die Kondensation der Derivate der Formeln II und III in einem polaren Lösungsmittel oder in einem aprotischen Lösungsmittel bei einer Temperatur zwischen 80 und 100°C in Gegenwart eines Akzeptors für die im Verlaufe der Reaktion gebildete Wasserstoffsaure durchgeführt wird.

## Claims

**Claims for the following Contracting States: AT, BE, CH, DE, FR, GB, IT, LI, LU, NL, SE**

1. Benzopyrrolidinone compounds of the general formula I:

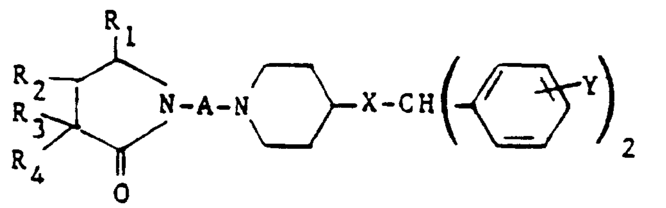

(I)

wherein :

– $R_1$, $R_2$, $R_3$ and $R_4$ have the following meanings:

**a)** $R_1$ and $R_2$, together with the carbon-carbon bond of the heterocycle to which they are attached, represent a benzene nucleus that is optionally substituted by one or more halogen atoms, such as fluorine or chlorine, or by one or more trifluoromethyl, alkyl or alkoxy radicals each having from 1 to 4 carbon atoms, or by methylenedioxy; and, simultaneously, $R_3$ and $R_4$, which are the same or different, each represents a hydrogen atom, a straight-chain or branched alkyl radical having from 1 to 4 carbon atoms, or a cycloalkyl radical having 5 or 6 carbon atoms;

or

**b)** $R_1$ represents a hydrogen atom, $R_4$ represents a single bond,
and, simultaneously, $R_2$ and $R_3$, together with the carbon-carbon bond of the heterocycle to which they are attached, represent a benzene nucleus that is optionally substituted by one or more halogen atoms, such as fluorine or chlorine, or by one or more trifluoromethyl, alkyl or alkoxy radicals each having from 1 to 4 carbon atoms, or by methylene-dioxy;

so as to form structures belonging to the indole and isoindole family of the following type:

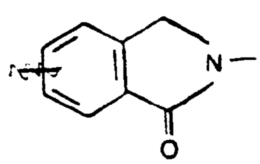

and

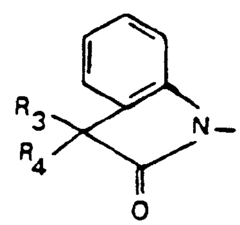

– A is a straight or branched hydrocarbon chain having from 2 to 6 carbon atoms that is optionally substituted by a hydroxy radical;

– X represents a single bond or an oxygen or sulphur atom;

and

– Y represents a hydrogen or halogen atom or an alkyl or alkoxy radical each having from 1 to 5 carbon atoms.

2. Salts of the compounds of claim 1 with biologically tolerable acids.

3. 1-(4-benzhydryloxypiperidinoethyl)-oxindole according to claim 1.

4. 2-(4-benzhydryloxypiperidinoethyl)-isooxindole according to claim 1.

5. A process for the preparation of the compounds of claim 1, characterised in that

– a compound of the general formula II :

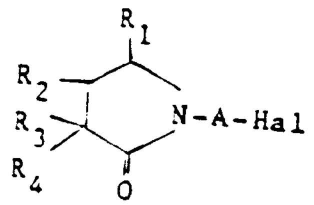

(II)

wherein :

- $R_1$, $R_2$, $R_3$, $R_4$ and A are as defined in claim 1, and
- Hal represents a chlorine or bromine atom,

is condensed with a compound of the general formula III:

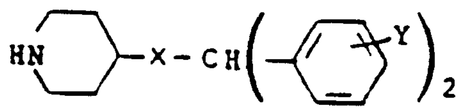

(III)

wherein X and Y are as defined in claim 1.

6. A preparation process according to claim 5, characterised in that the condensation of compounds II and III is carried out in a polar solvent or in an aprotic solvent, at a temperature of from 80 to 100°C, in the presence of an acceptor of the hydracid formed during the course of the reaction.

7. Pharmaceutical compositions containing a compound according to claims 1 to 4 as active ingredient, together with appropriate pharmaceutical excipients.

8. Pharmaceutical compositions according to claim 7 in a form suitable especially for the treatment of bronchospastic disorders and inflammations in the ENT sphere.

**Claims for the Contracting States: ES, GR**

1. A process for the preparation of benzopyrrolidinone compounds of the general formula I :

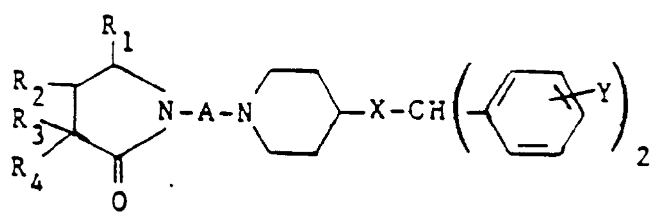

(I)

wherein :

- $R_1$, $R_2$, $R_3$ and $R_4$ have the following meanings:

**a)** $R_1$ and $R_2$, together with the carbon-carbon bond of the heterocycle to which they are attached, represent a benzene nucleus that is optionally substituted by one or more halogen atoms, such as fluorine or chlorine, or by one or more trifluoromethyl, alkyl or alkoxy radicals each having from 1 to 4 carbon atoms, or by methylenedioxy;
and, simultaneously, $R_3$ and $R_4$, which are the same or different, each represents a hydrogen atom, a straight-chain or branched alkyl radical having from 1 to 4 carbon atoms, or a cycloalkyl radical having 5 or 6 carbon atoms;
or
**b)** $R_1$ represents a hydrogen atom, $R_4$ represents a single bond,
and, simultaneously, $R_2$ and $R_3$, together with the carbon-carbon bond of the heterocycle to which they are attached, represent a benzene nucleus that is optionally substituted by one or more halogen atoms, such as fluorine or chlorine, or by one or more trifluoromethyl, alkyl or alkoxy radicals each

having from 1 to 4 carbon atoms, or by methylenedioxy;
so as to form structures belonging to the indole and isoindole family of the following type:

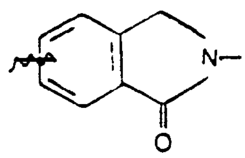

and

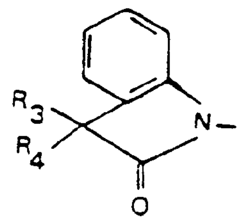

– A is a straight or branched hydrocarbon chain having from 2 to 6 carbon atoms that is optionally substituted by a hydroxy radical;
– X represents a single bond or an oxygen or sulphur atom;
and
– Y represents a hydrogen or halogen atom or an alkyl or alkoxy radical each having from 1 to 5 carbon atoms,

and their salts with biologically tolerable acids, characterised in that
– a compound of the general formula II :

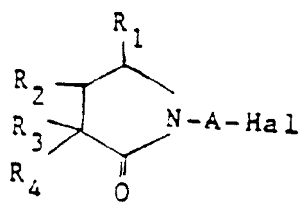

(II)

wherein :
– $R_1$, $R_2$, $R_3$, $R_4$ and A are as defined above, and
– Hal represents a chlorine or bromine atom,
is condensed with a compound of the general formula III:

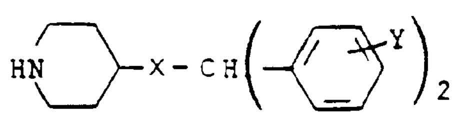

(III)

wherein X and Y are as defined above,
and, if desired, the products of formula I so obtained are treated with biologically tolerable mineral or organic acids to yield the corresponding addition salts.

2. A preparation process according to claim 1, characterised in that the condensation of compounds II and III is carried out in a polar solvent or in an aprotic solvent, at a temperature of from 80 to 100°C, in the presence of an acceptor of the hydracid formed during the course of the reaction.